(19) Europäisches Patentamt
European Patent Office
Office européen des brevets

(11) EP 1 258 237 A2

(12) # DEMANDE DE BREVET EUROPEEN

(43) Date de publication:
**20.11.2002 Bulletin 2002/47**

(51) Int Cl.[7]: **A61K 7/135**

(21) Numéro de dépôt: **02291065.7**

(22) Date de dépôt: **26.04.2002**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **16.05.2001 FR 0106462**

(71) Demandeur: **L'OREAL**
**75008 Paris (FR)**

(72) Inventeurs:
- **Legrand, Frédéric**
  **92400 Courbevoie (FR)**
- **Millequant, Jean-Marie**
  **94100 Saint-Maur des Fosses (FR)**

(74) Mandataire: **Miszputen, Laurent**
**L'Oreal,**
**Francine Arnaud - DPI,**
**6, rue Bertrand Sincholle**
**92585 Clichy Cédex (FR)**

(54) **Composition pulvérulente pour la décoloration des fibres kératiniques humaines**

(57) L'invention concerne une composition pulvérulente pour la décoloration des fibres kératiniques humaines, et en particulier les cheveux, comprenant au moins un sel peroxygéné, et en outre l'association de (i) au moins un polymère amphiphile non-ionique et/ou anionique comportant au moins une chaîne grasse, et (ii) moins de 10% en poids d'un agent particulier réduisant le taux de poussière choisi dans le groupe formé par la γ-butyrolactone, le diméthyl isosorbide, le diisopropyl isosorbide, les esters des monoalcools inférieurs saturés linéaires ou ramifiés en $C_3$-$C_6$ avec des acides bifonctionnels en $C_2$-$C_{24}$ ou avec des acides gras monofonctionnels en $C_{12}$-$C_{24}$, ladite composition ne pouvant être mélangée qu'avec un oxydant ne titrant pas plus de 40 volumes en peroxyde d'hydrogène afin qu'elle puisse donner une composition de décoloration homogène, stable, et d'application précise et aisée, et pour qu'elle puisse produire une décoloration des cheveux homogène et ne laissant pas les cheveux rêches.

EP 1 258 237 A2

## Description

La présente invention concerne une composition pulvérulente pour la décoloration des fibres kératiniques humaines, et plus particulièrement des cheveux, comprenant, dans un milieu approprié pour la décoloration, au moins un sel peroxygéné, et outre l'association de (i) au moins un polymère amphiphile non-ionique et/ou anionique comportant au moins une chaîne grasse, et (ii) moins de 10% en poids d'un agent particulier réduisant le taux de poussière. L'invention concerne également l'utilisation de ladite poudre pour la préparation d'une composition destinée à la décoloration des fibres kératiniques humaines, le procédé de préparation de la dite poudre et le procédé de décoloration des fibres kératiniques la mettant en oeuvre.

La décoloration des fibres kératiniques humaines et plus particulièrement des cheveux se fait par oxydation du pigment "mélanine" aboutissant à la solubilisation et l'élimination partielle ou totale de ce pigment.
Pour décolorer les cheveux, on utilise généralement des poudres décolorantes contenant un réactif peroxygéné tel que les persulfates, perborates et percarbonates, d'ammonium
ou de métaux alcalins, que l'on associe au moment de l'emploi à une composition aqueuse de peroxyde d'hydrogène. Les sels peroxygénés et le peroxyde d'hydrogène étant relativement stables en milieu acide, il est souvent nécessaire de les activer à pH basique pour obtenir une formation adéquate d'oxygène. Il est donc usuel d'ajouter aux poudres décolorantes, des composés alcalins tels que l'urée, les silicates, phosphates, ou carbonates de métaux alcalins ou alcalino-terreux, et en particulier les métasilicates de métaux alcalins, ou des agents précurseurs d'ammoniac comme les sels d'ammonium .

En cosmétique capillaire, cette composition aqueuse de peroxyde d'hydrogène ne doit pas titrer plus de 40 volumes.

Cependant, en salon de coiffure, afin d'augmenter les performances décolorantes des compositions pulvérulentes de décoloration, certains coiffeurs pourraient être tentés de faire usage de solutions d'eau oxygénée qui ne sont pas formulées pour un usage cosmétique et qui titrent plus de 40 volumes.

Ces pratiques seraient évidemment dangereuses et nuiraient au confort et à la sécurité du client ; aussi la Demanderesse a donc recherché des moyens pour empêcher ces pratiques éventuelles.
La Demanderesse a donc recherché une nouvelle poudre décolorante qui ne peut pas s'utiliser avec des compositions aqueuses d'eaux oxygénées à forts volumes (supérieurs à 40 volumes).

On sait que les poudres décolorantes ont tendance à former de la poussière durant leur manutention, leur transport et leur stockage; étant donné que les composés qui les composent (persulfates, silicates alcalins) sont corrosifs, irritants pour les yeux, les voies respiratoires et les muqueuses, on a déjà proposé de réduire le taux de poussière en déposant sur la poudre, par un procédé de pulvérisation, divers composés substantiellement insolubles dans l'eau tels que par exemple des huiles ou des cires liquides, lesquels en enrobant les particules de la poudre, les agglomèrent en des particules de taille plus grande. Le brevet EP-560088 décrit un tel procédé.
Pour réduire le taux de poussière, la demanderesse a antérieurement également proposé d'utiliser des polymères, anhydres, liquides et solubles dans l'eau à température ambiante, tels que ceux choisis parmi les polyéthylèneglycols ou les polypropylèneglycols (brevet français N° 2 716 804), ou ceux linéaires séquencés bloc et/ou statistique du type polyoxyéthylène/polyoxypropylène (brevet EP-663 205). Dans le brevet américain N°-5866107, pour réduire le taux de poussière, on a proposé des composés tels que la $\gamma$-butyrolactone, le diméthyl isosorbide, le diisopropyl isosorbide, ou des diesters d'alkyles en $C_3$-$C_6$.

Par ailleurs, pour localiser le produit de décoloration à l'application sur les cheveux afin qu'il ne coule pas sur le visage ou en dehors des zones que l'on se propose de décolorer, il est d'usage d'épaissir ou gélifier les compositions de décoloration avec des épaississants traditionnels tels que des polymères épaississants hydrosolubles comme les dérivés de cellulose, les dérivés d'amidon, l'acide polyacrylique réticulé, les alginates ou encore avec des silices épaississantes.

Ces épaississants traditionnels engendrant une chute de la viscosité des compositions décolorantes au cours du temps, la Demanderesse a plus récemment proposé, dans le brevet français 2 788 974, d'utiliser un système épaississant capable d'assurer le maintien d'une viscosité élevée pendant la durée nécessaire à obtenir la décoloration souhaitée, et consistant à associer l'épaississant hydrosoluble classique à un polymère amphiphile non ionique comportant au moins une chaîne grasse.

Par ailleurs, comme le traitement de décoloration est souvent agressif et conduit à de mauvaises propriétés cosmétiques des cheveux tels qu'un démêlage difficile, un toucher désagréable ou des cheveux rêches et ternes, et surtout à une dégradation des fibres, la Demanderesse a proposé dans le brevet français N° 2 788 976 de limiter significativement cette dégradation en employant une association de polymères amphiphiles non ioniques et/ou anioniques et de polymères substantifs cationiques ou amphotères.

Voici maintenant, qu'après d'importantes recherches menées sur la question, la Demanderesse a découvert une nouvelle poudre décolorante stable au stockage, susceptible de n'être mélangée qu'avec des compositions aqueuses de peroxyde d'hydrogène ne titrant pas plus de 40 volumes, pour pouvoir former des compositions de décoloration

prêtes à l'emploi homogènes et stables, dont l'application est aisée, se localisant bien sans couler ni diffuser sur les zones de la chevelure que l'on ne souhaite pas décolorer, et qui engendrent des décolorations puissantes et homogènes tout en ne laissant pas les cheveux rêches.
La demanderesse a en effet constaté que les mélanges de cette nouvelle poudre décolorante selon l'invention avec des compositions aqueuses de peroxyde d'hydrogène titrant plus de 40 volumes étaient hétérogènes et instables, ne permettaient pas une application facile et suffisamment précise sans coulures, et diffusaient sur les zones de la chevelure que l'on ne souhaitait pas décolorer; en outre, ils ont conduit à des décolorations capillaires hétérogènes en laissant les cheveux rêches.

La nouvelle poudre décolorante selon la présente invention est anhydre et non volatile, c'est à dire dépourvue de poussière (ou fines), et autrement dit, que la distribution granulométrique de ses particules est telle que le taux pondéral des particules qui ont une taille inférieure ou égale à 65 microns (taux de fines) est généralement inférieur ou égal à 5%, de préférence inférieur à 2% et plus particulièrement inférieur à 1%.

Ces découvertes sont à la base de la présente invention.

La présente invention a ainsi pour objet une composition pulvérulente pour la décoloration des fibres kératiniques humaines, et plus particulièrement des cheveux, comprenant, dans un milieu approprié pour la décoloration, au moins un sel peroxygéné, et en outre l'association de (i) au moins un polymère amphiphile non-ionique et/ou anionique comportant au moins une chaîne grasse, et (ii) moins de 10% en poids d'un agent réduisant le taux de poussière choisi dans le groupe formé par la γ-butyrolactone, le diméthyl isosorbide, le diisopropyl isosorbide, les esters des acides bifonctionnels en $C_2$-$C_{24}$ ou des acides gras monofonctionnels en $C_{12}$-$C_{24}$ avec des monoalcools inférieurs saturés linéaires ou ramifiés en $C_3$-$C_6$.
De préférence, selon l'invention, on utilise moins de 5% et plus particulièrement entre 0,5 et 2% dudit agent réduisant le taux de poussière.

Elle a pour autre objet l'utilisation de ladite composition pulvérulente pour la préparation d'une composition prête à l'emploi destinée à la décoloration des fibres kératiniques humaines et plus particulièrement des cheveux, par mélange au moment de l'emploi, de la dite poudre avec une composition aqueuse de peroxyde d'hydrogène ne titrant pas plus de 40 volumes.

Par "composition prête à l'emploi" on entend, au sens de l'invention, la composition destinée à être appliquée telle quelle sur les fibres kératiniques, c'est à dire qu'elle résulte du mélange extemporané de la poudre et de la composition aqueuse de peroxyde d'hydrogène.
Par anhydre, on entend, au sens de l'invention, une poudre dont la teneur en eau est inférieure à 5%, de préférence inférieure à 1% et plus particulièrement inférieure à 0,5% en poids.

L'invention vise également un procédé de décoloration des fibres kératiniques humaines, et plus particulièrement des cheveux utilisant la composition de décoloration prête à l'emploi telle que décrite selon l'invention.

Mais d'autres caractéristiques, aspects, objets et avantages de l'invention apparaîtront encore plus clairement à la lecture de la description et des exemples qui suivent.

<u>Polymères amphiphiles non ioniques comportant au moins une chaîne grasse</u>

Ils sont choisis de préférence parmi :

- **(1)** les celluloses modifiées par des groupements comportant au moins une chaîne grasse ; on peut citer à titre d'exemple :

  - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, comme le produit NATROSOL PLUS GRADE 330 CS (alkyles en $C_{16}$) vendu par la société AQUALON, ou le produit BERMOCOLL EHM 100 vendu par la société BEROL NOBEL,
  - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.

-**(2)** les hydroxypropylguars modifiés par des groupements comportant au moins une chaîne grasse en $C_8$ à $C_{22}$, tel que le produit JAGUAR XC-95/3 (chaîne alkyle en $C_{14}$) vendu par la société RHODIA, le produit ESAFLOR HM 22 (chaîne alkyle en $C_{22}$) vendu par la société LAMBERTI, les produits RE210-18 (chaîne alkyle en $C_{14}$) et RE205-1 (chaîne alkyle en $C_{20}$) vendus par la société RHONE POULENC.

- **(3)** les copolymères de vinyl pyrrolidone et de monomères hydrophobes à chaîne grasse dont on peut citer à titre d'exemple :

- les produits ANTARON V216 ou GANEX V216 (copolymère vinylpyrrolidone / hexadécène) vendu par la société I.S.P.
- les produits ANTARON V220 ou GANEX V220 (copolymère vinylpyrrolidone / eicosène) vendu par la société I.S.P.

- **(4)** les copolymères de méthacrylates ou d'acrylates d'alkyles en $C_1$-$C_6$ et de monomères amphiphiles comportant au moins une chaîne grasse.

- **(5)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

- **(6)** les polymères à squelette aminoplaste éther possédant au moins une chaîne grasse, tels que les composés PURE THIX proposés par la société SUD-CHEMIE.

- **(7)** les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom. Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut citer le Ser-ad FX 1100 de la société SERVO DELDEN qui est un copolymère dénommé sous la désignation I.N.C.I. européenne et américaine "Steareth-100/PEG-136/H.M.D.I. Copolymer".
On peut aussi utiliser le Rhéolate 205 à fonction urée vendu par la société RHEOX ou encore les Rhéolates 208 , 204 ou 212, ainsi que l'Acrysol RM 184.
On peut également citer le produit ELFACOS T210 à chaîne alkyle en $C_{12-14}$ et le produit ELFACOS T212 à chaîne alkyle en $C_{18}$ de chez AKZO.
Les polyéthers polyuréthanes utilisables selon l'invention sont en particulier ceux décrits dans l'article de G. Fonnum, J. Bakke et Fk. Hansen - Colloid Polym. Sci 271, 380.389 (1993).
On préfère en particulier les polyéthers polyuréthanes comportant au moins une chaîne grasse en $C_{10}$ à $C_{20}$, et les hydroxypropylguars modifiés par des groupements comportant au moins une chaîne grasse en $C_8$ à $C_{22}$.

Polymères amphiphiles anioniques comportant au moins une chaîne grasse

Les polymères amphiphiles anioniques comportant au moins une chaîne grasse utilisables selon la présente invention sont des polymères réticulés ou non réticulés, comprenant :

- des motifs hydrophiles dérivés d'un ou de plusieurs monomères à insaturation éthylénique portant une fonction acide carboxylique libre, ou une fonction sulfonique libre ou partiellement ou totalement neutralisée, et
- des motifs hydrophobes dérivés d'un ou de plusieurs monomères à insaturation éthylénique portant une chaîne latérale hydrophobe, et éventuellement
- des motifs de réticulation dérivés d'un ou plusieurs monomères polyinsaturés.

- **(I)** Le ou les monomères à insaturation éthylénique portant une fonction acide carboxylique sont choisis parmi l'acide éthacrylique, l'acide méthacrylique et l'acide acrylique, de préférence parmi l'acide méthacrylique, l'acide

acrylique et des mélanges de ceux-ci.
Le ou les monomères à insaturation éthylénique portant une chaîne latérale hydrophobe peuvent être (i) des esters d'acides carboxyliques insaturés et d'alcools gras, ou (ii) des éthers d'allyle et d'alcools gras.

(i) Les esters d'acides carboxyliques insaturés et d'alcools gras sont choisis par exemple parmi les éthacrylates, méthacrylates et/ou acrylates d'alkyles en $C_{10-30}$, de préférence en $C_{12-22}$. Ils englobent par exemple l'acrylate de lauryle, l'acrylate de stéaryle, l'acrylate de décyle, l'acrylate d'isodécyle, l'acrylate de dodécyle, et les méthacrylates correspondants, à savoir le méthacrylate de lauryle, le méthacrylate de stéaryle, le méthacrylate de décyle, le méthacrylate d'isodécyle, et le méthacrylate de dodécyle.
(ii) Les éthers allyliques d'alcools gras formant les motifs hydrophobes des polymères amphiphiles anioniques de la présente invention correspondent à la formule (I) suivante :

$$CH_2 = C\ R'\ CH_2\ O\ B_n\ R \qquad (I)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comportant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryle($C_{18}$).
Ledit monomère réticulant est un composé comportant au moins deux doubles liaisons polymérisables non ocnjuguées l'une avec l'autre. On peut citer à tire d'exemple le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.
Des polymères amphiphiles anioniques du type décrit ci-dessus sont décrits et préparés, par exemple dans les brevets US-3 915 921 et US-4 509 949 (copolymères d'acide (méth)acrylique et de (méth)acrylates d'alkyles en $C_{10-30}$, ou dans le brevet EP-0216479 B2 (copolymères d'acide (méth)acrylique et d'éthers allyliques d'alcools gras).

On peut citer à titre d'exemples de polymères préférés :

- les polymères réticulés d'acide acrylique et de méthacrylate d'alkyle en $C_{10-30}$, tels que le CARBOPOL ETD-2020 commercialisé par la société GOODRICH;
- les polymères réticulés d'acide acrylique et d'acrylate d'alkyle en $C_{10-30}$, tels que les polymères commercialisés sous les dénominations CARBOPOL 1382, PEMULEN TR1, PEMULEN TR2 par la société GOODRICH;
- le terpolymère acide méthacrylique/acrylate d'éthyle/méthacrylate de stéaryle oxyéthyléné (55/35/10);
- le terpolymère acide (méth)acrylique/acrylate d'éthyle/méthacrylate de béhényle oxyéthyléné 25 OE, et
- le terpolymère réticulé acide méthacrylique/acrylate d'éthyle/stéareth-10 allyl éther. **-(II)** les polymères amphiphiles comportant comme motifs hydrophiles au moins un monomère à insaturation éthylénique à groupement sulfonique, sous forme libre ou partiellement ou totalement neutralisée et au moins une partie hydrophobe sont par exemple décrits dans les demandes de brevets français de la demanderesse N°-0016954 et 0100328 dont le contenu fait partie intégrante de la présente invention.

On peut notamment citer plus particulièrement parmi eux :
le copolymère acide acrylamido-2-méthyl-2-propane-sulfonique(AMPS)/n-dodécylacrylamide neutralisé par la soude, le copolymère réticulé par du méthylène-bis-acrylamide constitué de 75% en poids de motifs AMPS neutralisés par $NH_3$ et de 25% en poids de motifs acrylate de GENAPOL T-250, le copolymère réticulé par du méthacrylate d'allyle constitué de 90% en poids de motifs AMPS neutralisés par $NH_3$ et de 10% en poids de motifs méthacrylate de GENAPOL T-250, ou le copolymère réticulé par du méthacrylate d'allyle constitué de 80% en poids de motifs AMPS neutralisés par $NH_3$ et de 20% en poids de motifs méthacrylate de GENAPOL T-250.
Dans la poudre décolorante selon l'invention, le ou les polymères amphiphiles non ioniques et/ou anioniques comportant au moins une chaîne grasse peuvent être présents dans une proportion pondérale comprise entre 0,01 et 30% et de préférence entre 0,01 et 15% par rapport au poids total de la poudre décolorante.

<u>Agents réduisant le taux de poussière</u>

Parmi les esters de monoalcools inférieurs saturés linéaires ou ramifiés en $C_3$-$C_6$, avec des acides bifonc-

tionnels en $C_2$-$C_{24}$ ( ces derniers pouvant être linéaires ou ramifiés, saturés ou insaturés, on préfère, selon la présente invention, utiliser les di-esters isopropyliques desdits acides et plus particulièrement des acides adipique, oxalique ou sébacique (adipate de di-isopropyle, oxalate de di-isopropyle, sébacate de di-isopropyle).

Parmi les esters de monoalcools inférieurs saturés linéaires ou ramifiés en $C_3$-$C_6$, avec des acides gras monofonctionnels en $C_{12}$-$C_{24}$ ( ces derniers pouvant être linéaires ou ramifiés, saturés ou insaturés), on préfère, selon la présente invention, utiliser les esters isopropyliques de ces acides gras comme notamment et par exemple le palmitate d'isopropyle. Lesdits esters d'acides gras monofonctionnels en $C_{12}$-$C_{24}$ peuvent être notamment choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates, etc...

Parmi tous ces agents réduisant le taux de poussière selon la présente invention, on peut citer, à titre d'exemple, le produit vendu sous la dénomination ISO ADIPAT par la société DRAGOCO, qui est un adipate de di-isopropyle, le produit vendu sous la dénomination ISOPROPYL PALMITATE par la société COGNIS qui est un palmitate d'isopropyle, le produit vendu sous la dénomination DUB.DIS par la société STEARINERIE DUBOIS qui est un sébacate de di-isopropyle.

Dans la poudre décolorante selon l'invention, le ou lesdits agents réduisant le taux de poussière sont présents dans une proportion pondérale inférieure à 10% par rapport au poids total de la poudre décolorante, de préférence inférieure à 5%, et plus particulièrement comprise entre 0,5 et 2%.

Sels peroxygénés.

Comme indiqué ci-dessus, ils sont choisis parmi les persulfates, les perborates et les percarbonates d'ammonium ou de métaux alcalins ainsi que le peroxyde de magnésium et les mélanges de ces composés.
On utilise de préférence les persulfates, et parmi ceux-ci principalement les persulfates de sodium et de potassium.
Dans la poudre décolorante selon l'invention, le ou les sels peroxygénés peuvent être présents dans une proportion pondérale comprise entre 20 et 70% et de préférence entre 30 et 60% par rapport au poids total de ladite poudre.

Agents alcalins

Ces agents alcalins déjà décrits ci-dessus (urée, silicates, phosphates, ou carbonates de métaux alcalins ou alcalino-terreux, et en particulier métasilicates de métaux alcalins, ou agents précurseurs d'ammoniac comme les sels d'ammonium), peuvent être présents dans la poudre décolorante dans une proportion pondérale comprise entre environ 0,01 et 40% et de préférence entre environ 0,1 et 30% par rapport au poids total de la poudre décolorante.
Selon une alternative, ils peuvent être présents dans une composition aqueuse à mélanger au moment de l'emploi à la composition aqueuse de peroxyde d'hydrogène.

Polymères épaississants hydrosolubles

En outre, la poudre décolorante selon la présente invention peut contenir de tels polymères.
Selon l'invention, ils englobent tous les polymères hydrosolubles synthétiques ou d'origine naturelle utilisés classiquement dans le domaine cosmétique et différents des polymères amphiphiles non ioniques et/ou anioniques comportant au moins une chaîne grasse de la présente invention.
Comme exemples de polymères synthétiques, on peut citer, la polyvinylpyrrolidone, l'acide polyacrylique, le polyacrylamide, l'acide poly-2-acrylamidopropanesulfonique non réticulé comme par exemple le produit vendu sous la dénomination SIMUGEL EG par la société SEPPIC, l'acide poly-2-acrylamido-2-méthylpropane sulfonique réticulé, l'acide poly-2-acrylamido-2-méthylpropane sulfonique réticulé et partiellement neutralisé par l'ammoniaque vendu sous la marque HOSTACERIN AMPS par la société CLARIANT, des mélanges à effet épaississant synergique de l'acide poly-2-acrylamido-2-méthylpropane sulfonique non réticulé avec des éthers d'hydroxyalkylcellulose ou avec des poly(oxyde d'éthylène) tel qu'ils sont décrits dans le brevet US-4540510, ou des mélanges à effet épaississant synergique d'un acide poly(méth)acrylamido-alkyl($C_1$-$C_4$)-sulfonique de préférence réticulé avec un copolymère réticulé de l'anhydride maléique et d'un alkyl($C_1$-$C_5$)vinyléther tels que le mélange HOSTACERIN AMPS / STABILEZE QM (de la société ISF) et tels qu'ils sont décrits dans la demande de brevet français de la demanderesse N°-0014416.

Les polymères épaississants d'origine naturelle utilisables selon la présente invention, sont de préférence des polymères comportant au moins un motif sucre, à savoir : les gommes de guar non-ioniques; les gommes de biopolysaccharides d'origine microbienne telles que les gommes de Scléroglucane ou de Xanthane; les gommes issues d'exudats végétaux telles que les gommes Arabique, Ghatti, Karaya, Tragacanthe, Carrageenane, Agar et Caroube; les pectines; les alginates; les amidons; les hydroxyalkyl($C_1$-$C_6$)celluloses et carboxyalkyl($C_1$-$C_6$)celluloses.

Par "motif sucre", on désigne au sens de la présente invention, une portion monosaccharidique (i.e. monosaccharide ou oside ou sucre simple) ou une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides (homopolyosides ou hétéropolyosides)]. Les unités saccharidiques peuvent être en outre substituées par des radicaux alkyle, ou hydroxyalkyle, ou alcoxy, ou acyloxy, ou carboxyle, les radicaux alkyle comportant de 1 à 4 atomes de carbone.

Les gommes de guar non ioniques peuvent être modifiées ou non modifiées.

Les gommes de guar non modifiées sont par exemple les produits vendus sous la dénomination GUARGEL D/15 par la société GOODRICH, VIDOGUM GH 175 par la société UNIPECTINE, et sous les dénominations MEYPRO-GUAR 50 et JAGUAR C par la société MEYHALL.

Les gommes de guar non-ioniques modifiées sont notamment modifiées par des groupements hydroxyalkyle en $C_1$-$C_6$. Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHONE POULENC (MEYHALL) ou sous la dénomination GALACTASOL 4H4FD2 par la société AQUALON.

Les gommes de biopolysaccharides d'origine microbienne telles que les gommes de scléroglucane ou de xanthane, les gommes issues d'exudats végétaux telles que les gommes Aabique, gomme Ghatti, gomme Karaya, Tragacanthe, Carrageenane, Agar et Caroube, les hydroxyalkylcelluloses et les carboxyméthylcelluloses, les pectines, les alginates et les amidons sont bien connus de l'homme de l'art et décrits notamment dans l'ouvrage de Robert L. DAVIDSON intitulé "Handbook of Water soluble gums and resins" édité chez Mc Graw Hill Book Company (1980).

Parmi ces gommes, les scléroglucanes sont représentés par les produits vendus sous la dénomination ACTIGUM CS par la société SANOFI BIO INDUSTRIES et en particulier ACTIGUM CS 11 et sous la dénomination AMIGEL par la société ALBAN MULLER INTERNATIONAL. D'autres scléroglucanes tels que celui traité au glyoxal dans la demande de brevet français N°2633940 peuvent également être utilisés.

Les Xanthanes sont représentés par les produits vendus sous les dénominations KELTROL, KELTROL T, KELTROL TF, KELTROL BT, KELTROL RD, KELTROL CG par la société NUTRASWEET KELCO, ou sous les dénominations RHODICARE S, RHODICARE H par la société RHODIA CHIMIE.

Parmi les dérivés d'amidon, on peut citer par exemple le produit vendu sous la dénomination PRIMOGEL par la société AVEBE.

Les hydroxyalkyl($C_1$-$C_6$)celluloses sont plus particulièrement des hydroxyéthylcelluloses telles que celles vendues sous les dénominations CELLOSIZE QP3L, CELLOSIZE QP4400H, CELLOSIZE QP30000H, CELLOSIZE HEC30000A, CELLOSIZE POLYMER PCG10, par la société AMERCHOL, ou NATROSOL 250HHR, NATROSOL 250MR, NATROSOL 250M, NATROSOL 250HHXR, NATROSOL 250HHX, NATROSOL 250HR, NATROSOL HX, par la société HERCULES, ou encore TYLOSE H1000 par la société HOECHST. Les hydroxyalkyl($C_1$-$C_6$)celluloses sont également plus particulièrement des hydroxypropylcelluloses comme les produits vendus sous les dénominations KLUCEL EF, KLUCEL H, KLUCEL LHF, KLUCEL MF, KLUCEL G, par la société AQUALON.

Parmi les carboxyalkyl($C_1$-$C_6$)celluloses, on utilise de préférence la carboxyméthylcellulose dont on peut citer les produits vendus sous les dénominations BLANOSE 7M8/SF, BLANOSE RAFFINEE 7M, BLANOSE 7LF, BLANOSE 7MF, BLANOSE 9M31F, BLANOSE 12M31XP, BLANOSE 12M31P, BLANOSE 9M31XF , BLANOSE 7H, BLANOSE 7M31, BLANOSE 7H3SXF, par la société AQUALON, ou encore AQUASORB A500 et AMBERGUM 1221, par le société HERCULES, ou encore CELLOGEN HP810A et CELLOGEN HP6HS9, par la société MONTELLO, ou encore PRIMELLOSE par la société AVEBE.

Lorsqu'ils ils sont présents dans les compositions pulvérulentes de la présente invention, les polymères épaississants hydrosolubles sont présents dans une proportion pondérale inférieure ou égale à 5% par rapport au poids total de ladite composition et de préférence inférieure ou égale à 3%.

Autres adjuvants

Pour éviter que la poudre décolorante anhydre ne se dégrade à l'humidité ambiante pendant le stockage, il est possible d'y ajouter des absorbeurs d'humidité tels que par exemple des silices comme la silice colloïdale, la silice pyrogénée à caractère hydrophobe ou hydrophile, et dans une proportion pondérale inférieure ou égale à 3% par rapport au poids total de la poudre décolorante. La poudre décolorante selon l'invention peut en outre contenir des

charges telles que des argiles, des liants tels que la vinylpyrrolidone, des lubrifiants comme les stéarates de polyol ou les stéarates de métaux alcalins ou alcalino-terreux, ainsi que des agents de contrôle du dégagement d'oxygène tels que le carbonate ou l'oxyde de magnésium, des agents colorants ou des agents matifiants comme les oxydes de titane ou encore des agents tensioactifs anioniques, non ioniques ou amphotères.

La poudre décolorante selon l'invention peut aussi contenir des polymères conditionneurs cationiques ou amphotères anhydres bien connus de l'homme de l'art et décrits dans les brevets français N°-2788974 et 2788976 et tels que décrits ci-après.

Polymères cationiques

Au sens de la présente invention, l'expression "polymère cationique" désigne tout polymère contenant des groupements cationiques et/ou des groupements ionisables en groupements cationiques.
Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.
Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.
Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :

**(1)** Les homopolymères ou copolymères dérivés d'esters ou d'amides acryliques ou méthacryliques et comportant au moins un des motifs de formules (I), (II), (III) ou (IV) suivantes:

**(I)**
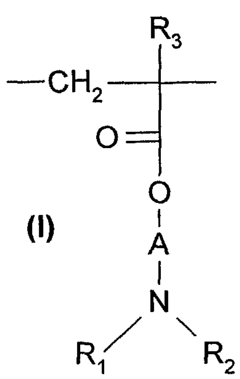
**(II)**
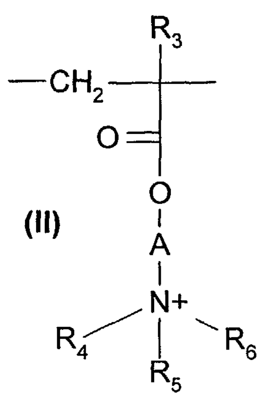
**(III)**
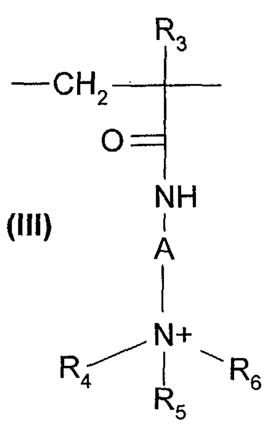

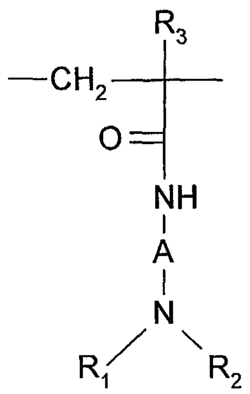

(IV)

dans lesquelles:

$R_3$ , identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$, identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :

- les copolymères d'acrylamide et de diméthylaminoéthyl méthacrylate quaternisé au sulfate de diméthyle ou avec un hologénure de diméthyle;
- les copolymères d'acrylamide et de chlorure de méthacryloyloxyéthyltriméthyl-ammonium décrits par exemple dans la demande de brevet EP-A-080976;
- le copolymère d'acrylamide et de méthosulfate de méthacryloyloxyéthyltriméthyl-ammonium;
- les copolymères vinylpyrrolidone / acrylate ou méthacrylate de dialkylaminoalkyle quaternisés ou non. Ces polymères sont décrits en détail dans les brevets français 2.077.143 et 2.393.573;
- les terpolymères méthacrylate de diméthyl amino éthyle/ vinylcaprolactame/ vinylpyrrolidone;
- les copolymère vinylpyrrolidone / méthacrylamidopropyl diméthylamine;
- et les copolymères vinylpyrrolidone / méthacrylamide de diméthylaminopropyle quaternisés.

**(2)** Les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium .

**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylmidopropyl triméthylammonium, de diméthyl-diallylammonium.

**(4)** Les polysaccharides cationiques décrits plus particulièrement dans les brevets US 3 589 578 et 4 031 307 tel

que les gommes de guar contenant des groupements cationiques trialkylammonium. On utilise par exemple des gommes de guar modifiées par un sel (par ex. chlorure) de 2,3-époxypropyl triméthylammonium.

**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .

**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363. Parmi ces dérivés, on peut citer plus particulièrement les polymères acide adipique/diméthylaminohydroxypropyl/diéthylène triamine.

**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) :

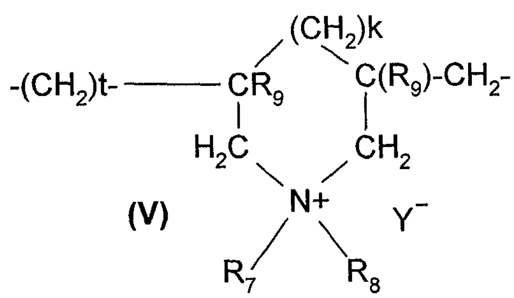

**(V)**

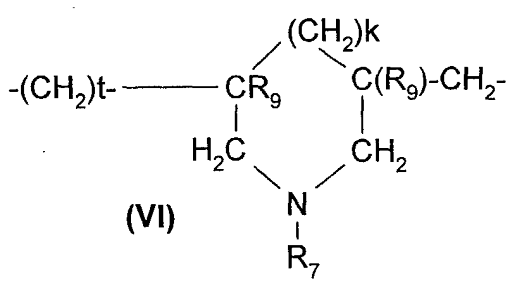

**(VI)**

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$ indépendamment l'un de l'autre désignent de préférence un groupement alkyle ayant de 1 à 4 atomes de carbone ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

**(10)** Le polymère de diammonium quaternaire contenant des motifs récurrents répondant à la formule :

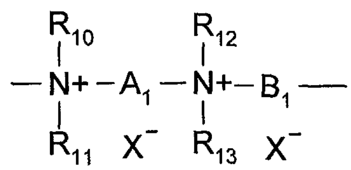

**(VII)**

formule (VII) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$, $R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH-$R_{14}$-D où $R_{14}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
$X^-$ désigne un anion dérivé d'un acide minéral ou organique;
A1, $R_{10}$ et $R_{12}$ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - $(CH_2)$n-CO-D-OC-$(CH_2)$n- dans lequel n est compris entre 1 et 100 et de préférence entre 1 et 50, et D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH- .

De préférence, $X^-$ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (VIII) suivante:

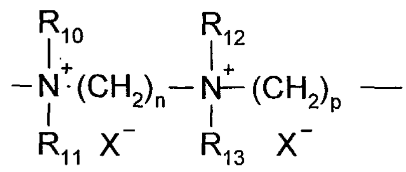

**(VIII)**

dans laquelle $R_{10}$, $R_{11}$, $R_{12}$ et $R_{13}$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

**(11)** Les polymères de poly(ammonium quaternaire) constitués de motifs récurrents de formule (IX) :

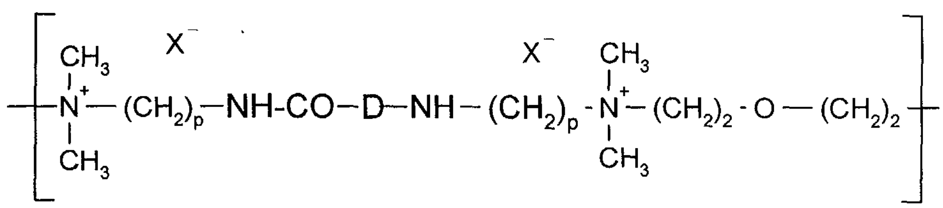

**(IX)**

dans laquelle p désigne un nombre entier variant de 1 à 6 environ, D peut être nul ou peut représenter un groupement $-(CH_2)_r$ -CO- dans lequel r désigne un nombre égal à 4 ou à 7, X- est un anion ;
De tels polymères peuvent être préparés selon les procédés décrits dans les brevets U.S.A. n° 4 157 388, 4 702 906, 4 719 282. Ils sont notamment décrits dans la demande de brevet EP-A-122 324.

**(12)** Les polymères quaternaires de vinylpyrrolidone et de vinylimidazole.

**(13)** Les polyamines comme le produit référencé sous le nom de " POLYETHYLENEGLYCOL (15) TALLOW POLYAMINE " dans le dictionnaire CTFA.

**(14)** Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/ chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de "SALCARE® SC 92" par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de "SALCARE® SC 95 " et "SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
On peut également citer le copolymère acrylate de sodium / chlorure d'acrylamidopropyl trimethyl ammonium.
Le composé vinylique peut être également un sel de dialkyldiallylammonium tel que le chlorure de diméthyldiallylammonium.

(2) Les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
Les comonomères basiques préférés sont des méthacrylates d'aminoéthyle, de butyl aminoéthyle, de N,N'-diméthylaminoéthyle, de N-tertio-butylaminoéthyle.

(3) Les polyaminoamides réticulés et alcoylés partiellement ou totalement dérivant de polyaminoamides de formule générale :

$$-\!\!\left[ CO - R_{19} - CO - Z \right]\!\!- \qquad \textbf{(X)}$$

dans laquelle $R_{19}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine

bis primaire ou bis secondaire, et Z désigne un radical d'une polyalkylène-polyamine bis-primaire, mono ou bis-secondaire et de préférence représente :

a) dans les proportions de 60 à 100 moles %, le radical

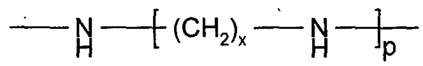

**(XI)**

où x=2 et p=2 ou 3, ou bien x=3 et p=2 ce radical dérivant de la diéthylène triamine, de la triéthylène tétraamine ou de la dipropylène triamine;

b) dans les proportions de 0 à 40 moles % le radical (XI) ci-dessus, dans lequel x=2 et p=1 et qui dérive de l'éthylènediamine, ou le radical dérivant de la pipérazine :

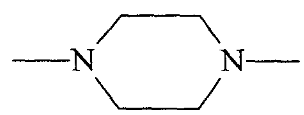

c) dans les proportions de 0 à 20 moles % le radical $-NH-(CH_2)_6-NH-$ dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique. Les alcanes sultones utilisées dans l'alcoylation sont de préférence la propane ou la butane sultone, les sels des agents d'alcoylation sont de préférence les sels de sodium ou de potassium.

(4) Les polymères comportant des motifs zwittérioniques de formule :

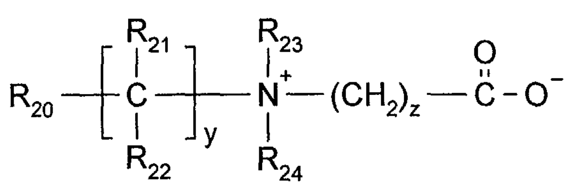

**(XII)**

dans laquelle $R_{20}$ désigne un groupement insaturé polymérisable tel qu'un groupement acrylate, méthacrylate, acrylamide ou méthacrylamide, y et z représentent un nombre entier de 1 à 3, $R_{21}$ et $R_{22}$ représentent un atome d'hydrogène, méthyle, éthyle ou propyle, $R_{23}$ et $R_{24}$ représentent un atome d'hydrogène ou un radical alkyle de telle façon que la somme des atomes de carbone dans $R_{23}$ et $R_{24}$ ne dépasse pas 10.

Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.

A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.

(5) les polymères dérivés du chitosane comportant des motifs monomères répondant aux formules (XIII), (XIV), (XV) suivantes :

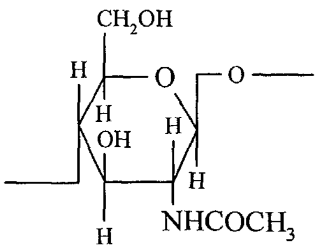

(XIII)

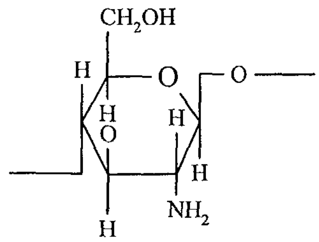

(XIV)

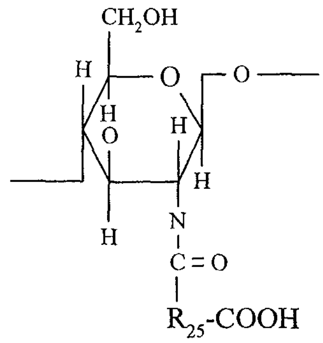

(XV)

le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), $R_{25}$ représente un radical de formule :

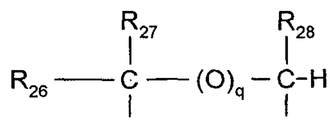

dans laquelle q désigne zéro ou 1 ;

si q=0, $R_{26}$, $R_{27}$ et $R_{28}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{26}$, $R_{27}$ et $R_{28}$ étant dans ce cas un atome d'hydrogène ;

ou si q=1, $R_{26}$, $R_{27}$ et $R_{28}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane.

(7) Les polymères répondant à la formule générale (XI) tels que ceux décrits par exemple dans le brevet français 1 400 366 :

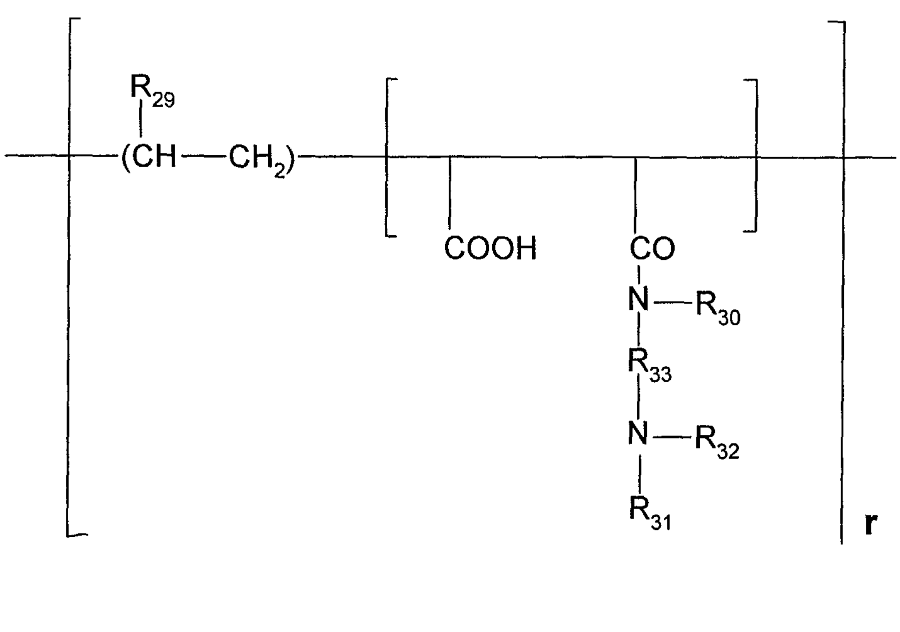

(XVI)

dans laquelle $R_{29}$ représente un atome d'hydrogène, un radical $CH_3O$, $CH_3CH_2O$, phényle, $R_{30}$ désigne l'hydrogène ou un radical alkyle inférieur tel que méthyle, éthyle, $R_{31}$ désigne l'hydrogène ou un radical alkyle inférieur tel que méthyle, éthyle, $R_{32}$ désigne un radical alkyle inférieur tel que méthyle, éthyle ou un radical répondant à la formule : $-R_{33}-N(R_{31})_2$, $R_{33}$ représentant un groupement $-CH_2-CH_2-$ , $-CH_2-CH_2-CH_2-$, $-CH_2-CH(CH_3)-$, $R_{31}$ ayant les significations mentionnées ci-dessus, ainsi que les homologues supérieurs de ces radicaux et contenant jusqu'à 6 atomes de carbone,
r est tel que le poids moléculaire est compris entre 500 et 6000000 et de préférence entre 1000 et 1000000.

(8) Des polymères amphotères du type -D-X-D-X- choisis parmi:

a) les polymères obtenus par action de l'acide chloracétique ou le chloracétate de sodium sur les composés comportant au moins un motif de formule :

-D-X-D-X-D- **(XVII)**

où D désigne un radical

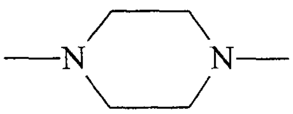

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

-D-X-D-X- **(XVIII)**

où D désigne un radical

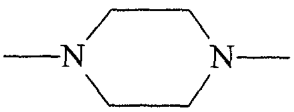

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi tous les polymères cationiques ou amphotères utilisables selon la présente invention, on préfère notamment :

(i)parmi les cationiques :

- l'homopolymère de chlorure de diméthyldiallylammonium vendu sous la dénomination MERQUAT 100DRY par la société MERCK;
- les copolymères de chlorure de diméthyldiallylammonium et d'acrylamide vendus sous la dénomination MERQUAT 2200 par la société CALGON;
- les polymères de type poly(ammonium quaternaire) préparés et décrits dans le brevet français 2 270 846, constitués de motifs récurrents de formules (W) et (U) suivantes :

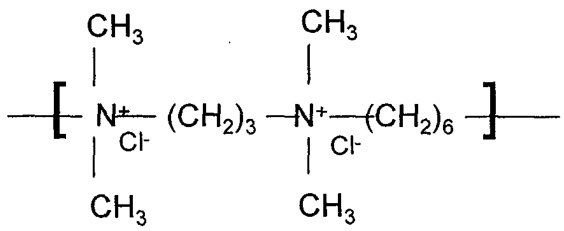

**(W)**

et notamment ceux dont le poids moléculaire, déterminé par chromatographie par perméation de gel, est compris entre 9500 et 9900;

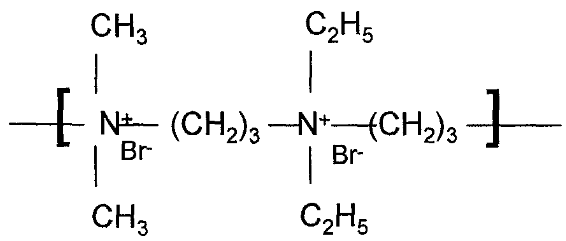

**(U)**

et notamment ceux dont le poids moléculaire, déterminé par chromatographie par perméation de gel, est d'environ 1200;
- les polymères de type poly(ammonium quaternaire) de la famille (11) et de formule (IX) dans laquelle $X^-$ désigne le Chlore, et notamment ceux dont la masse moléculaire moyenne en poids est inférieure à 100 000, de préférence inférieure ou égale à 50 000;

(ii)parmi les polymères amphotères:

- le copolymère chlorure de diméthyldiallylammonium/acide acrylique (80/20) commercialisé sous la dénomination MERQUAT 280 DRY par la société CALGON (dénomination CTFA : POLYQUATERNIUM 22);
- le copolymère chlorure de diméthyldiallylammonium/acide acrylique (95/5) commercialisé sous la dénomination MERQUAT 295 DRY par la société CALGON (dénomination CTFA : POLYQUATERNIUM 22);
- le copolymère de chlorure de méthacrylamidopropyltrimonium, d'acide acrylique et d'acrylate d'éthyle, commercialisé sous la dénomination MERQUAT 2001 par la société CALGON (dénomination CTFA : POLYQUATERNIUM 47); et
- le terpolymère acrylamide/chlorure de diméthyldiallylammonium/acide acrylique, commercialisé sous la dénomination MERQUAT PLUS 3330 DRY par la société CALGON (dénomination CTFA : POLYQUATERNIUM 39).

Lorsqu'ils ils sont présents dans les compositions pulvérulentes de la présente invention, les polymères cationiques et/ou amphotères sont présents dans une proportion pondérale inférieure ou égale à 20% par rapport au poids total de ladite composition et de préférence inférieure ou égale à 8%.

<u>Préparation de la poudre décolorante selon l'invention</u>

Selon la présente invention, on prépare la poudre décolorante par simple addition de la quantité désirée d'agent réduisant le taux de poussière décrit ci-dessus à un mélange poudreux constitué de sel(s) peroxygéné(s),

d'agent(s) alcalin(s), de polymère(s) amphiphile(s) anionique(s) et/ou non-ionique(s), et éventuellement de polymère épaississant hydrosoluble et de silice.

Composition aqueuse de peroxyde d'hydrogène

La composition aqueuse de peroxyde d'hydrogène selon la présente invention ne titre pas plus de 40 volumes, et son titre peut varier de 2 à 40 volumes.

Elle est formulée exclusivement pour un usage cosmétique et contient de préférence de façon connue à cet effet :

(i) soit au moins un agent tensioactif de type quelconque c'est à dire non ionique, anionique, cationique ou amphotère ou zwittérionique mais dont au moins un anionique ou non-ionique,
dans une proportion pondérale totale comprise entre 0,1 et 10% et de préférence entre 0,1 et 5% par rapport au poids total de la composition aqueuse de peroxyde d'hydrogène;

(ii) soit au moins l'association de deux agents tensioactifs non-ioniques de HLB [Hydrophilic Lipophilic Balance] différents et dont au moins l'un est inférieur ou égal à 5 selon l'échelle de GRIFFIN*,
dans une proportion pondérale totale comprise entre 1,5 et 40% et de préférence entre 1,5 et 20% par rapport au poids total de la composition aqueuse de peroxyde d'hydrogène;
[GRIFFIN* définie dans la publication J. Soc. Cosm. Chem. 1954 (Volume 5), pages 249-256, ou la HLB déterminée par voie expérimentale et telle que décrite dans l'ouvrage des auteurs F. PUISIEUX et M. SEILLER, intitulé "GALENICA 5 : Les systèmes dispersés - Tome I - Agents de surface et émulsions - Chapitre IV - Notions de HLB et de HLB critique, pages 153-194 - paragraphe 1.1.2. Détermination de HLB par voie expérimentale, pages 164-180].

(iii) soit au moins l'association d'un agent tensioactif non ionique de HLB [Hydrophilic Lipophilic Balance] inférieur ou égal à 5 selon l'échelle de GRIFFIN* et d'un agent tensioactif anionique,
dans une proportion pondérale totale comprise entre 1 et 30% et de préférence entre 1,5 et 15% par rapport au poids total de la composition aqueuse de peroxyde d'hydrogène;

(iv) soit au moins un polymère épaississant quelle que soit sa nature chimique, dans une proportion pondérale comprise entre 0,1 et 10% et de préférence entre 0,1 et 5% par rapport au poids total de la composition aqueuse de peroxyde d'hydrogène;
ledit polymère étant éventuellement associé à un agent tensioactif de type quelconque, dans une proportion pondérale comprise entre 0,1 et 10% et de préférence entre 0,1 et 5% par rapport au poids total de la composition aqueuse de peroxyde d'hydrogène.

Ce polymère épaississant est par exemple choisi parmi les polymères épaississants hydrosolubles déjà décrits ci-dessus, les polymères amphiphiles comportant au moins une chaîne grasse et dont la nature ionique n'est pas critique, c'est à dire qu'ils peuvent être de type non-ionique ou anionique comme ceux décrits ci-dessus pour la composition pulvérulente non volatile, ou encore de type cationique ou amphotère comme ceux décrits ci-après.
La composition de peroxyde d'hydrogène étant aqueuse, il va de soi que les polymères amphiphiles de type non-ionique ou anionique peuvent en outre se présenter sous forme de solutions ou de dispersions à base d'eau.
C'est par exemple le cas des polyéthers polyuréthanes non-ioniques à chaîne grasse comme :

- l'ACULYN 44 et l'ACULYN 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)];
- le produit DW 1206B de chez ROHM & HAAS à chaîne alkyle en $C_{20}$ et à liaison uréthanne, proposé à 20 % en matière sèche dans l'eau;
- les produits Rhéolate 255, Rhéolate 278 et Rhéolate 244 vendus par la société RHEOX, ou les produits DW 1206F et DW 1206J proposés par la société ROHM & HAAS.

C'est également le cas de polymères amphiphiles anioniques à chaîne grasse comme par exemple:

- les terpolymères réticulés d'acide méthacrylique, d'acrylate d'éthyle, de polyéthylèneglycol (10 OE) éther d'alcool

stéarylique (Steareth 10), vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10);

- le terpolymère acide méthacrylique/acrylate de méthyle/diméthyl métaisopropényl benzyl isocyanate d'alcool béhényle éthoxylé (40 OE) en dispersion aqueuse à 25% décrit dans l'exemple 3 du brevet EP-A-0173109.

Parmi les polymères amphiphiles comportant au moins une chaîne grasse et de type amphotère, on peut citer notamment ceux décrits et préparés dans la demande de brevet WO9844012, et plus particulièrement les copolymères acide acrylique/chlorure de (méth)acrylamidopropyl triméthyl ammonium/ méthacrylate de stéaryle.

Parmi les polymères amphiphiles comportant au moins une chaîne grasse et de type cationique, on peut citer notamment :

- ceux choisis parmi les dérivés de cellulose quaternisée, dont en particulier,
  - les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
  - les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

  Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

  On peut indiquer comme exemples d'alkylhydroxyéthyl-celluloses quaternisées à chaînes grasses en $C_8$-$C_{30}$, les produits QUATRISOFT LM 200, QUATRISOFT LM-X 529-18-A, QUATRISOFT LM-X 529-18B (alkyle en $C_{12}$) et QUATRISOFT LM-X 529-8 (alkyle en $C_{18}$) commercialisés par la société AMERCHOL et les produits CRODACEL QM, CRODACEL QL (alkyle en $C_{12}$) et CRODACEL QS (alkyle en C18) commercialisés par la société CRODA ;
- ceux choisis parmi les polyacrylates à groupements latéraux aminés non cycliques, quaternisés ou non, qui possèdent par exemple des groupements hydrophobes du type stéareth 20 (alcool stéarylique polyoxyéthyléné(20)).

  Comme exemples de polyacrylates à chaînes latérales aminées, on peut citer les polymères 8781- 121B ou 9492-103 proposés par la société NATIONAL STARCH.
- ceux choisis dans la famille des polyuréthanes associatifs cationiques décrite par la demanderesse dans la demande de brevet français n°-0009609; elle peut être représentée par la formule générale (XIX) suivante :

$$R\text{-}X\text{-}(P)_n\text{-}L\text{-}(Y)_m\text{-}L'\text{-}(P')_p\text{-}X'\text{-}R' \qquad \text{(XIX)}$$

dans laquelle :

R et R', identiques ou différents, représentent un groupement hydrophobe ou un atome d'hydrogène ;

X et X', identiques ou différents, représentent un groupement comportant une amine tertiaire ou quaternaire portant ou non un groupement hydrophobe, ou encore le groupement L'' ;

L, L' et L'',identiques ou différents, représentent un groupement dérivé d'un diisocyanate ;

P et P', identiques ou différents, représentent un groupement comportant une amine tertiaire ou quaternaire portant ou non un groupement hydrophobe ;

Y représente un groupement hydrophile ;

n, m, et p valent chacun indépendamment des autres entre 0 et 1000 ; la molécule contenant au moins une fonction amine tertiaire ou quaternaire et au moins un groupement hydrophobe.

Une famille préférée de polyuréthanes associatifs cationiques est celle correspondant à la formule (XIX) décrite ci-dessus et dans laquelle :

R et R' représentent tous les deux indépendamment un groupement hydrophobe,

X, X' représentent chacun un groupe L",

n et pvalent entre 1 et 1000 et

L, L', L", P, P', Y et m ont la signification indiquée ci-dessus.

Une autre famille préférée de polyuréthanes associatifs cationiques est celle correspondant à la formule (XIX) ci-dessus dans laquelle :

R et R' représentent tous les deux indépendamment un groupement hydrophobe,

X et X'représentent tous les deux indépendamment un groupement comportant une amine quaternaire,

n et p valent zéro, et

L, L', Y et m ont la signification indiquée ci-dessus.

La masse moléculaire des polyuréthanes associatifs cationiques est comprise de préférence entre 400 et 10 000 et plus particulièrement entre 1000 et 5000.
Par groupement hydrophobe, on entend au sens de l'invention, un radical ou un polymère dont la chaîne est hydrocarbonée, saturée ou non, linéaire ou ramifiée, et peut contenir un ou plusieurs hétéroatomes tels que P, O, N, S, ou dont la chaîne est perfluorée ou siliconée. Lorsque le groupement hydrophobe désigne un radical hydrocarboné, il comporte au moins 10 atomes de carbone.
Préférentiellement, le groupement hydrocarboné provient d'un composé monofonctionnel.
A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.
Lorsque X et/ou X' désignent un groupement comportant une amine tertiaire ou quaternaire, X et/ou X' peuvent représenter l'une des formules suivantes :

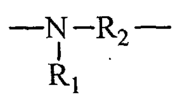 ou 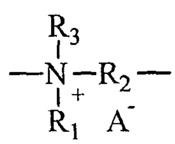 pour X

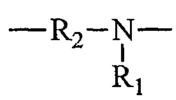 ou 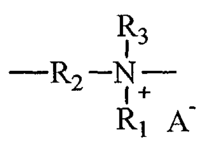 pour X'

dans lesquelles :

$R_2$ représente un radical alkylène ayant de 10 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;

$R_1$ et $R_3$, identiques ou différents, désignent un radical alkyle ou alcényle en $C_1$-$C_{30}$, linéaire ou ramifié, un radical aryle, l'un au moins des atomes de carbone pouvant être remplacé par un hétéroatome choisi parmi N, S, O, P ;

A-est un contre-ion physiologiquement acceptable.

Les groupements L, L' et L" représentent un groupe de formule :

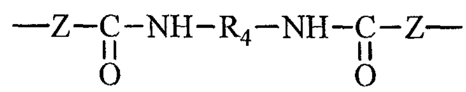

dans laquelle :

Z représente -O-, -S- ou -NH- ; et

$R_4$ représente un radical alkylène ayant de 10 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, et P.

Les groupements P et P', comprenant une fonction amine tertiaire ou quaternaire, peuvent représenter au moins l'une des formules suivantes :

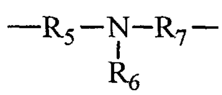

ou

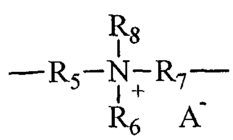

ou

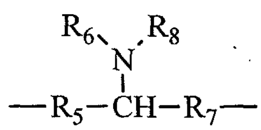

ou

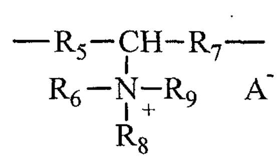

ou

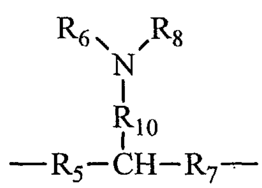

ou

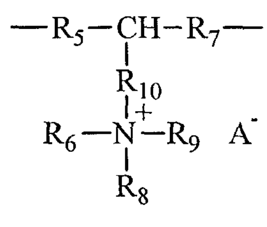

dans lesquelles :

$R_5$ et $R_7$ ont les mêmes significations que $R_2$ ;

$R_6$, $R_8$ et $R_9$ ont les mêmes significations que $R_1$ et $R_3$ ;

R10représente un groupe alkylène, linéaire ou ramifié, éventuellement insaturé et pouvant contenir un ou plusieurs hétéroatomes choisis parmi N, O, S et P, et $A^-$ est un contre-ion physiologiquement acceptable.

En ce qui concerne la signification de Y, on entend par groupement hydrophile, un groupement hydrosoluble

polymérique ou non.
A titre d'exemple, on peut citer, lorsqu'il ne s'agit pas de polymère, le diéthylèneglycol et l'hexaéthylèneglycol.
Lorsqu'il s'agit d'un polymère, on peut citer à titre d'exemple, les polyéthers, les polyesters sulfonés, les polyamides sulfonés, ou un mélange de ces polymères. A titre préférentiel, le polymère hydrophile est un polyéther et notamment un poly(oxyde d'éthylène) ou poly(oxyde de propylène).

Le polyuréthane associatif cationique de formule (XIX) selon l'invention est formé à partir de différents composés possédant chacun au moins un hydrogène labile. On fait réagir ces différents groupements les uns avec les autres de manière successive afin de former les différentes séquences du polymère.

Un premier type de composés entrant dans la préparation du polymère de formule (XIX) de l'invention est un composé comportant au moins un motif amine tertiaire ou quaternaire. Ce composé peut être multifonctionnel, mais préférentiellement le composé est difonctionnel, c'est-à-dire que selon un mode de réalisation préférentiel de l'invention, ce composé comporte deux atomes d'hydrogène labiles portés par exemple par une fonction hydroxyle, amine secondaire, amine tertiaire ou thiol. On peut également utiliser un mélange de composés multifonctionnels et difonctionnels dans lequel le pourcentage de composés multifonctionnels est faible.
Comme indiqué précédemment, ce composé peut comporter plus d'un motif amine tertiaire ou quaternaire. Il s'agit alors d'un polymère portant une répétition du motif amine tertiaire ou quaternaire.

Ce type de composés peut être représenté par l'une des formules suivantes :

$$HZ\text{-}(P)_n\text{-}ZH,$$

ou

$$HZ\text{-}(P')_p\text{-}ZH$$

dans lesquelles Z, P, P', n et p sont tels que définis plus haut.
A titre d'exemple, on peut citer la N-méthyldiéthanolamine, la N-tert-butyl-diéthanolamine, la N-sulfoéthyldiéthanolamine.

Un deuxième composé entrant dans la préparation du polymère de formule (XIX) selon l'invention est un diisocyanate correspondant à la formule :

$$O=C=N\text{-}R_4\text{-}N=C=O$$

dans lequelle $R_4$ est tel que défini plus haut.
A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polymère de formule (XIX) selon l'invention est un composé hydrophobe destiné à la formation de la séquence hydrophobe terminale du polymère de formule (XIX). Cette séquence hydrophobe peut être formée à partir d'un composé comportant un hydrogène labile porté par exemple par une fonction hydroxyle, amine primaire ou secondaire, ou thiol. Il s'agit de préférence d'un composé monofonctionnel. La partie fonctionnelle de ce composé peut contenir un motif amine tertiaire ou quaternaire. La partie hydrophobe de ce composé peut ou non être un polymère.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné $\alpha$-hydroxylé.
La séquence hydrophobe du composé de formule (XIX) peut également résulter de la réaction de quaternisation de l'amine tertiaire du composé comportant au moins un motif amine tertiaire. Ainsi, le groupement hydrophobe est porté par l'agent quaternisant. Il s'agit alors d'un composé de type RQ ou R'Q, dans lequel R et R' sont tels que définis plus haut et Q désigne un groupe tel qu'un halogénure, un sulfate etc.

Le polyuréthane associatif cationique de l'invention peut en outre comprendre une séquence hydrophile.
Cette séquence est apportée par un quatrième type de composés entrant dans la préparation du polymère. Ce composé peut être multifonctionnel. Il est de préférence difonctionnel. On peut également avoir un mélange où le pourcentage en composé multifonctionnel est faible.
Les parties fonctionnelles, possédant l'hydrogène labile, peuvent être par exemple des fonctions alcool, amine primaire ou secondaire, ou thiol. Ce composé peut être un polymère terminé aux extrémités des chaînes par l'une de ces fonctions possédant l'hydrogène labile.

A titre d'exemple, on peut citer, lorsqu'il ne s'agit pas de polymère, le diéthylèneglycol et l'hexaéthylèneglycol. Lorsqu'il s'agit d'un polymère, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le polymère préparé à partir des composés définis plus haut, est un polyuréthane associatif cationique de formule (XIX) selon la présente invention. Ce polymère est soluble ou dispersible dans l'eau et augmente de manière spectaculaire la viscosité de la solution aqueuse dans laquelle il est dissous ou dispersé.

La séquence hydrophile notée Y dans la formule (XIX) est facultative. En effet, les motifs amine quaternaire peuvent suffire à apporter la solubilité nécessaire pour ce type de polymère dans une solution aqueuse.

Tensioactifs non-ioniques oxyalkylénés de HLB inférieure ou égale à 5.

Parmi eux, on peut citer de manière non limitative :

- les alkylphénols oxyéthylénés ayant au plus 2 moles d'OE,
- les condensats OE/OP dont le rapport OP/OE est supérieur à 0,71,
- les huiles végétales oxyéthylénées ayant au plus 5 moles d'OE,
- les alcools gras oxyéthylénés ayant au plus 2 moles d'OE,
- les alcools gras et les esters d'alcools gras comportant de préférence de 8 à 18 atomes de carbone,
- les amides gras comportant de préférence de 8 à 18 atomes de carbone.

Tensioactif(s) de type anionique, non-ionique, amphotère ou zwittérionique, cationique :

Tensioactifs anioniques

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkyl ($C_6$-$C_{24}$) sulfosuccinates, les alkyl($C_6$-$C_{24}$) éthersulfosuccinates, les alkyl($C_6$-$C_{24}$) amidesulfosuccinates ; les alkyl ($C_6$-$C_{24}$) sulfoacétates ; les acyl($C_6$-$C_{24}$) sarcosinates et les acyl($C_6$-$C_{24}$) glutamates. On peut également utiliser les esters d'alkyl($C_6$-$C_{24}$)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

Tensioactifs non ioniques :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine.

Tensioactifs amphotères ou zwittérioniques :

Les agents tensioactifs amphotères ou zwitterioniques peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_3$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes. Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_2\text{ -CONHCH}_2\text{CH}_2\text{ -N(R}_3\text{)(R}_4\text{)(CH}_2\text{COO}^-\text{)}$$

dans laquelle : $R_2$ désigne un radical alkyle d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;

$$R_2\text{'-CONHCH}_2\text{CH}_2\text{-N(B)(C)}$$

dans laquelle :

B représente -$CH_2CH_2OX'$, C représente -$(CH_2)_z$-Y', avec z = 1 ou 2,

X' désigne le groupement -$CH_2CH_2$-COOH ou un atome d'hydrogène

Y' désigne -COOH ou le radical -$CH_2$- CHOH - $SO_3H$

$R_2$' désigne un radical alkyle d'un acide $R_9$-COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid. A titre d'exemple on peut citer le cocoamphodiacetate commercialisé sous la dénomination commerciale MIRANOL® C2M concentré par la société RHODIA CHIMIE.

Tensioactifs cationiques :

Parmi les tensioactifs cationiques on peut citer en particulier (liste non limitative) : les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

La composition aqueuse de peroxyde d'hydrogène contient de préférence au moins un agent stabilisant choisi de préférence parmi les pyrophosphates des métaux alcalins ou alcalino-terreux, les stannates des métaux alcalins ou alcalino-terreux, la phénacétine ou les sels d'acides et d'oxyquinoléine, comme le sulfate d'oxyquinoléine. De manière plus avantageuse, on utilise au moins un stannate en association ou non à au moins un pyrophosphate.

La composition aqueuse de peroxyde d'hydrogène présente de préférence un pH inférieur à 7 .
Le pH acide garantit la stabilité du peroxyde d'hydrogène dans la composition.
Il peut être obtenu à l'aide d'agents acidifiants tels que par exemple l'acide chlorhydrique, l'acide acétique, l'acide éthydronique, l'acide phosphorique, l'acide lactique ou l'acide borique, et il peut être ajusté classiquement par ajout soit d'agents basifiants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

La composition aqueuse de peroxyde d'hydrogène peut encore contenir des agents conservateurs, des colorants, des parfums, des agents antimousse, et des agents séquestrants tels que par exemple l'acide éthylènediamine tétraacétique (EDTA) ou le Pentasodium Pentetate (dénomination CTFA).

Bien entendu, l'homme de l'art veillera à choisir le ou les éventuels composés complémentaires mentionnés ci-avant, de manière telle que les propriétés avantageuses attachées intrinsèquement à la composition pulvérulente de décoloration ou à la composition de décoloration prête à l'emploi selon l'invention ne soient pas, ou substantiellement pas, altérées par la ou les adjonctions envisagées.

Le pH de la composition de décoloration prête à l'emploi est généralement compris entre les valeurs 4 et 12. Il est de préférence compris entre 7 et 11,5 et encore plus préférentiellement entre 8 et 11.

De préférence, le procédé de décoloration selon l'invention consiste à mélanger au moment de l'emploi une composition pulvérulente telle que décrite selon la présente invention avec une composition aqueuse de peroxyde d'hydrogène ne titrant pas plus de 40 volumes et telle que décrite selon la présente invention, à appliquer la composition de décoloration prête à l'emploi ainsi obtenue sur la zone des fibres kératiniques humaines sèches ou humides à décolorer, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

La présente invention a aussi pour objet un dispositif à plusieurs compartiments, ou "kit", pour la décoloration des fibres kératiniques humaines et plus particulièrement des cheveux, caractérisé par le fait qu'il comporte au moins deux compartiments dont l'un contient une composition pulvérulente telle que décrite ci-dessus, et l'autre une composition aqueuse de peroxyde d'hydrogène ne titrant pas plus de 40 volumes.

La présente invention a aussi pour objet un procédé de préparation de ladite composition pulvérulente qui consiste à mélanger simplement à la température d'environ 25°C, le ou les agents réduisant le taux de poussière décrits ci-dessus au mélange préformé des autres composants de la composition pulvérulente (sel peroxygéné, polymère amphiphile non-ionique et/ou anionique comportant au moins une chaîne grasse, agent alcalin et autres adjuvants divers).

Des exemples concrets illustrant l'invention sont indiqués ci-après, sans pour autant présenter un caractère limitatif.

**EXEMPLE 1 :**

On a préparé la composition pulvérulente anhydre et non volatile pour la décoloration des cheveux (A) suivante (quantités exprimées en grammes) :

| | |
|---|---|
| Persulfate de Potassium..... | 48 |
| Persulfate de Sodium..... | 8 |
| Metasilicate de Sodium..... | 12 |
| Chlorure d'ammonium..... | 4.5 |
| Oxyde de Magnésium..... | 1 |
| Urée..... | 4 |
| EDTA (séquestrant)..... | 1 |
| Argile..... | 4.5 |
| Polymère amphiphile non-ionique à chaîne grasse: Ser-ad FX 1100 vendu par la Société Servo Delden..... | 3 |
| Polymère amphiphile non-ionique à chaîne grasse: Jaguar XC-95/3 vendu par la Société Rhodia..... | 1 |
| Polymère épaississant hydrosoluble: Alginate de Sodium..... | 2 |
| Tensio-actif anionique: Cétostéaryl sulfate de Sodium..... | 3 |
| Stéarate de Calcium..... | 2 |
| Oxyde de titane..... | 2 |
| Colorant | 0.2 |
| Adipate de di-isopropyle vendu sous la dénomination ISO ADIPAT par la société DRAGOCO..... | 2 |

1°/ Evaluation de la stabilité au stockage d'un échantillon de la composition (A)

On a évalué la stabilité au stockage dans le temps d'un échantillon de la composition A ci-dessus, en mesurant le titre en persulfates de Sodium et de Potassium de la composition, avant et après 2 mois de stockage à 45°C (respectivement T1 et T2). [Ces conditions de stockage accélérées permettent de prédire la stabilité de l'échantillon dans des conditions normales de stockage].
Le titre en persulfates de Sodium et de Potassium et exprimé en meq/g a été déterminé par dosage potentiel redox, en milieu acide, et en présence de Sulfate Ferreux, à l'aide d'une solution de Permanganate de Potassium.
On a donc déterminé la perte en persulfates de Sodium et de Potassium après 2 mois à 45°C (P) par le calcul suivant :

$$(P) = 100 * [(T1-T2) / T1]$$

Lorsque cette perte (P) est inférieure à 5 %, on considère que l'échantillon présentera une bonne stabilité dans des conditions normales de stockage.
Ainsi, on a étudié la stabilité au stockage de l'échantillon (A) après 2 mois à 45°C; les résultats ont été les suivants :

| | T1 (titre **avant** stockage 2 mois à 45°C) | T2 (titre **après** stockage 2 mois à 45°C) | Perte en Persulfates |
|---|---|---|---|
| Echantillon (A) | 4.20 meq/g | 4.15 meq/g | 1,2% |

On en conclut que la composition (A) pulvérulente pour la décoloration selon l'invention présente une très bonne stabilité au stockage.

2°/ Mesure de la granulométrie de la composition (A)

On a réalisé une granulométrie sur un échantillon de la composition (A) à l'aide d'un granulomètre RETSCH AS 200 DIGIT (Hauteur d'oscillation: 1.25 mm / Temps de tamisage : 5 mn).

| Diamètre des tamis en microns | Particules retenues par le tamis correspondant en % |
|---|---|
| 800 microns | 2 % |
| 400 microns | 29.5 % |
| 200 microns | 51.5 % |
| 125 microns | 15.5 % |
| 63 microns | 1.5 % |
| 32 microns | 0 % |
| Fond | 0 % |

La composition (A) selon l'invention n'a pas de particules fines dont le diamètre est inférieur à 63 microns.

3°/ Mélange avant emploi de la composition (A) avec une composition aqueuse stabilisée de peroxyde d'hydrogène.

On a comparé deux mélanges réalisés avant emploi :

- celui de la composition (A) avec une composition aqueuse (B) stabilisée de peroxyde d'hydrogène à 30 volumes (9%), conforme à l'invention avec,
- celui de cette même composition (A) avec une composition aqueuse (Bbis) stabilisée de peroxyde d'hydrogène

non formulée pour un usage cosmétique (110 volumes), non conforme à l'invention.

Les compositions (B) et (Bbis) sont données ci-après : (quantités exprimées en grammes %)

| | Composition (B) | Composition (Bbis) |
|---|---|---|
| Peroxyde d'hydrogène à 50 %......... | 18 (30 vol) | 66 (110 vol) |
| Tensio-actif non-ionique de HLB =1: Alcool cétostearylique.................. | 2.5 | |
| Tensio-actif non-ionique de HLB=2: Tridéceth-2 carboxamide MEA......... | 0.8 | |
| Tensio-actif non-ionique: Alcool cétostéarylique oxyéthylméné 30 OE....................................... | 0.6 | |
| Pentasodium Pentetate (séquestrant) | 0.05 | |
| Pyrophosphate tétrasodique........... | 0.03 | |
| Stannate de sodium.................... | 0.02 | |
| Acide phosphorique à 85 %........... | 0.4 | |
| Eau..............q.s.p...................... | 100 | 100 |

Les mélanges (A)(B) et (A)(Bbis) ont été réalisés en mélangeant 30 g de la composition pulvérulente non volatile (A) avec 45 g de la composition aqueuse stabilisée de peroxyde d'hydrogène (B) ou (Bbis).

<u>4°/ Résultats :</u>

Chaque mélange (A)(B) et (A)(Bbis) a été appliqué sur les cheveux pour les décolorer.

Le mélange (A)(Bbis) était hétérogène et instable. Il n'a pas permis une application facile et suffisamment précise sans coulures, et a diffusé sur les zones de la chevelure que l'on ne souhaitait pas décolorer. Il a conduit à une décoloration capillaire hétérogène, en laissant les cheveux rêches.

Le mélange (A)(B) était homogène, facilement applicable et suffisamment épais pour permettre une application précise sur les zones de la chevelure que l'on voulait décolorer. Il a permis d'obtenir une décoloration capillaire puissante et homogène tout en laissant les cheveux beaucoup moins rêches qu'avec le mélange (A)(Bbis).

On a également constaté que la composition (A) permettait des dilutions, plus importantes, avec la composition aqueuse stabilisée de peroxyde d'hydrogène (B) selon l'invention qu'avec la composition aqueuse de peroxyde d'hydrogène (Bbis) non formulée pour un usage cosmétique.

**<u>EXEMPLE 2:</u>**

1°/ On a préparé les compositions pulvérulentes anhydres et non volatiles pour la décoloration des cheveux (C), (D), (E), (F) et (G), suivantes : (quantités exprimées en grammes)

| COMPOSITIONS ⇒ | (C) | (D) | (E) | (F) | (G) |
|---|---|---|---|---|---|
| Persulfate de Potassium............ | 35 | 37.5 | 39.5 | 36 | 20 |
| Persulfate de Sodium............... | 10 | 5 | 5 | | 20 |
| Peroxyde de Magnésium à 20 %.. | | 2 | | 1.9 | 4.3 |
| Disilicate de Sodium................. | 12 | 24 | 25 | 22 | 25 |
| Métasilicate de Sodium............. | 12 | 1.3 | | 2 | |
| Chlorure d'ammonium.............. | 3 | 2.5 | 2.5 | 2 | 4 |
| Carbonate de Magnésium.......... | 5 | | | 4 | 1.5 |
| Oxyde de Magnésium............... | | | 0.3 | | |
| Urée...................................... | 3 | 4 | 2 | 3 | |
| EDTA (séquestrant)................. | 1 | 0.5 | 0.5 | 0.5 | 1 |
| Silice colloïdale....................... | | | | | 0.5 |
| Silice pyrogénée à caractère hydrophile.............................. | 2.5 | | | | |
| Silice pyrogénée à caractère | | | | | |

| | | | | | |
|---|---|---|---|---|---|
| hydrophobe.......................... | | | | 2.7 | |
| Argile.................................. | 3 | 4 | 4 | 3 | 3 |
| Polymère amphiphile non-ionique à chaîne grasse: Ser-ad FX 1100 de la Société Servo Delden......... | 3 | | | | 2 |
| Polymère amphiphile non-ionique à chaîne grasse: Jaguar XC-95/3 de la Société Rhodia................ | 2.8 | | 1 | | 2 |
| Polymère amphiphile anionique à chaîne grasse: Carbopol ETD2020 de la Société GOODRICH ........... | | 5 | 4 | 5 | |
| Polymère amphiphile anionique à chaîne grasse: CARBOPOL 1382 de la société GOODRICH........... | | | | | 2 |
| Polymère épaississant hydrosoluble: Gomme de Guar non ionique non modifiée (GUARGEL D/15 de la société GOODRICH).... | 2 | | | | 1.7 |
| Polymère épaississant hydrosoluble: PRIMOGEL de la société AVEBE........................ | | 3 | 3 | 0.5 | |
| Lauryl sarcosinate de sodium........ | 2 | | | | 0.5 |
| Cétostéaryl sulfate de sodium....... | 1 | 2 | 2 | 2 | 3 |
| Stéarate de Magnésium.............. | 3 | 3 | | 2 | |
| Stéarate de Calcium................. | | | 3 | | 2 |
| Polyvinylpyrrolidone................... | 2 | 2.8 | 3 | 2 | 2 |
| Oxyde de titane......................... | 2 | 1 | 1 | 1 | 2 |
| Colorant................................ | | 0.2 | | | 0.5 |
| Polymère conditionneur amphotère POLYQUATERNIUM 22 ........... | 0.2 | | 0.2 | | 0.5 |
| Parfum.................................. | | 0.2 | | 0.5 | |
| Palmitate d'isopropyle vendu sous la dénomination ISOPROPYL PALMITATE par la société COGNIS............................... | 9.5 | | | | |

| | | | | | |
|---|---|---|---|---|---|
| Sébacate de di-isopropyle vendu sous la dénomination DUB. DIS par la société STEARINERIE DUBOIS................................ | | 2 | | | |
| Diméthyl-isosorbide, vendu sous la dénomination ARLASOLVE DMI par la société UNICHEMA (ICI)..... | | | 2 | | |
| Adipate de di-isopropyle vendu sous la dénomination ISO ADIPAT par la société DRAGOCO........... | | | | 9.9 | 2.5 |

2°/ On a également préparé les compositions aqueuses stabilisées de peroxyde d'hydrogène (H), (I), (J), (K) et (L), suivantes :
(quantités exprimées en grammes)

| COMPOSITIONS ⇒ | (H) | (I) | (J) | (K) | (L) |
|---|---|---|---|---|---|
| Peroxyde d'hydrogène à 50 %....... | 18 (30 vol) | 24 (40 vol) | 18 (30 vol) | 12 (20 vol) | 18 (30 vol) |
| Tensio-actif non-ionique de HLB=1 Alcool cétostéarylique.................. | | | 2 | 1 | |
| Tensio-actif non-ionique de HLB=1 Alcool cétylique.......................... | | | 1 | 3 | |
| Tensio-actif non-ionique de HLB=5: Alcool cétylique oxyéthyléné 2 OE | 1.1 | | | | |
| Tensio-actif non-ionique: Alcool cétostéarylique oxyéthyléné 30 OE | 3 | | 0.5 | | 1 |
| Tensio-actif non-ionique: Alcool cétylique oxyéthyléné 10 OE........ | 2.2 | | 0.7 | | |
| Tensio-actif anionique: Cétéaryl sulfate de sodium...................... | | | | 0.5 | |
| Tensio-actif anionique: Lauryl sulfate de sodium...................... | | | | 0.2 | |
| Tensio-actif anionique: (C14/C16)oléfine sulfonate de sodium.................................... | | | 0.1 | 0.3 | 1 |
| Tensio-actif anionique: Cocoyl | | | | | |

| | | | | | |
|---|---|---|---|---|---|
| iséthionate de sodium................. | | | 0.1 | | |
| Polymère amphiphile anionique à chaîne grasse: ACULYN 22 de la Société Rohm & Haas.......... | 2 | | | | |
| Polymère épaississant hydrosoluble: SIMULGEL EG de la Société SEPPIC................ | | 2 | | | |
| Polymère épaississant hydrosoluble: HOSTACERIN AMPS de la Société CLARIANT... | | 3 | | | |
| Phosphate disodique.................. | | | 0.08 | | 0.05 |
| Pentasodium Pentetate (séquestrant)............................ | 0.05 | | | 0.06 | |
| Pyrophosphate tétrasodique......... | 0.03 | 0.04 | 0.03 | 0.04 | 0.02 |
| Stannate de sodium.................. | 0.01 | 0.02 | 0.02 | / | 0.02 |
| Parfum.................................... | | | 1 | 0.5 | |
| Agent antimousse : Simethicone.... | | | 0.5 | 0.6 | |
| Acide éthydronique.....q.s..pH...... | | 3.5 | | 2.5 | |
| Acide phosphorique à 85 % ............................q.s..pH...... | 3 | | 2.5 | | 2 |
| Eau déminéralisée............. | q.s.p.....100 | | | | |

3°/ On a ensuite préparé les compositions de décoloration prêtes à l'emploi (C)(H), (D)(L), (E)(J), (F)(K), et (G)(I), suivantes :

- 30g de la composition pulvérulente (C) + 60g de la composition de peroxyde d'hydrogène (H),
- 30g de la composition pulvérulente (D) + 40g de la composition de peroxyde d'hydrogène (L),
- 30g de la composition pulvérulente (E) + 45g de la composition de peroxyde d'hydrogène (J),
- 30g de la composition pulvérulente (F) + 40g de la composition de peroxyde d'hydrogène (K),
- 30g de la composition pulvérulente (G) + 40g de la composition de peroxyde d'hydrogène (I).

Les compositions de décoloration prêtes à l'emploi ainsi obtenues et conformes à l'invention, étaient homogènes, facilement applicables et suffisamment épaisses pour permettre une application précise sur les zones de la chevelure que l'on se proposait de décolorer. Elles ont permis d'obtenir des décolorations puissantes et homogènes, tout en ne laissant pas les cheveux rêches.

Par comparaison, des compositions de décoloration prêtes à l'emploi (C)(Bbis), (D)(Bbis), (E)(Bbis), (F)(Bbis) et (E)(Bbis) qui ont préparées à partir de 30g de chacune des compositions pulvérulentes (C) ou (D) ou (E) ou (F) ou (G) avec 30g de la composition de peroxyde d'hydrogène (Bbis) décrite au paragraphe 3°/ de l'exemple 1, étaient hétérogènes et trop instables. Elles n'ont pas permis une application facile et suffisamment précise sans coulures et ont diffusé sur les zones de la chevelure que l'on ne souhaitait pas décolorer. Elles ont conduit à des décolorations capillaires hétérogènes et ont laissé les cheveux rêches.

En outre, on a constaté que les compositions pulvérulentes de décoloration (C), (D), (E), (F) et (G) étaient

non volatiles et stables au stockage. En outre, elles ont permis des dilutions bien plus importantes avec les compositions aqueuses stabilisées de peroxyde d'hydrogène (H), (I), (J), (K) et (L), conformes à l'invention, qu'avec la composition aqueuse de peroxyde d'hydrogène (Bbis) non formulée pour un usage cosmétique.

## Revendications

1. Composition pulvérulente pour la décoloration des fibres kératiniques humaines, et plus particulièrement des cheveux, comprenant, dans un milieu approprié pour la décoloration, au moins un sel peroxygéné, **caractérisée par le fait qu'**elle contient en outre l'association de (i) au moins un polymère amphiphile non-ionique et/ou anionique comportant au moins une chaîne grasse, et (ii) moins de 10% en poids d'un agent réduisant le taux de poussière choisi dans le groupe formé par la γ-butyrolactone, le diméthyl isosorbide, le diisopropyl isosorbide, les esters de monoalcools inférieurs saturés linéaires ou ramifiés en $C_3$-$C_6$ avec des acides bifonctionnels en $C_2$-$C_{24}$ ou avec des acides gras monofonctionnels en $C_{12}$-$C_{24}$.

2. Composition selon la revendication 1, **caractérisée par le fait que** le ou les agents réduisant le taux de poussière sont présents en une quantité pondérale inférieure à 5% par rapport au poids total de la composition.

3. Composition selon la revendication 2, **caractérisée par le fait que** le ou les agents réduisant le taux de poussière sont présents en une quantité pondérale comprise entre 0,5 et 2% par rapport au poids total de la composition.

4. Composition selon l'une quelconque des revendications précédentes, **caractérisée par le fait que** le ou les agents réduisant le taux de poussière sont choisis parmi les di-esters isopropyliques des acides bifonctionnels en $C_2$-$C_{24}$.

5. Composition selon la revendication 4, **caractérisée par le fait que** le ou les agents réduisant le taux de poussière sont choisis parmi di-esters isopropyliques des acides adipique, oxalique et sébacique.

6. Composition selon l'une quelconque des revendications 1 à 3, **caractérisée par le fait que** le ou les agents réduisant le taux de poussière sont choisis parmi les esters isopropyliques des acides gras monofonctionnels en $C_{12}$-$C_{24}$.

7. Composition selon la revendication 6, **caractérisée par le fait que** l'agent réduisant le taux de poussière est le palmitate d'isopropyle.

8. Composition selon l'une quelconque des revendications précédentes, **caractérisée par le fait que** le polymère amphiphile non-ionique comportant au moins une chaîne grasse est choisi parmi les hydroxypropylguars modifiés par des groupements comportant au moins une chaîne grasse en $C_8$ à $C_{22}$, et les polyéthers polyuréthanes comportant au moins une chaîne grasse en $C_{10}$ à $C_{20}$.

9. Composition selon l'une quelconque des revendications 1 à 7, **caractérisée par le fait que** le polymère amphiphile anionique comportant au moins une chaîne grasse est choisi parmi les polymères réticulés d'acide acrylique et de (méth)acrylate d'alkyle en $C_{10-30}$.

10. Composition selon l'une quelconque des revendications précédentes, **caractérisée par le fait que** le ou les polymères amphiphiles non-ioniques et/ou anioniques comportant au moins une chaîne grasse sont utilisés en une quantité variant de 0,01 à 30% en poids du poids total de la composition.

11. Composition selon la revendication 10, **caractérisée par le fait que** lesdits polymères sont utilisés en une quantité variant de 0,01 à 15% en poids du poids total de la composition.

12. Composition selon l'une quelconque des revendications précédentes, **caractérisée par le fait qu'**elle contient en outre au moins un polymère hydrosoluble différent des polymères amphiphiles définis aux revendications 1, 8 à 11, qui est synthétique ou d'origine naturelle, dans une quantité inférieure ou égale à 5% en poids par rapport au poids total de la composition.

13. Composition selon l'une quelconque des revendications précédentes, **caractérisée par le fait qu'**elle contient en outre au moins une silice dans une quantité inférieure ou égale à 3% en poids par rapport au poids total de la composition.

**14.** Composition selon l'une quelconque des revendications précédentes, **caractérisée par le fait qu'**elle contient en outre au moins un polymère cationique et/ou amphotère dans une proportion pondérale inférieure ou égale à 20% par rapport au poids total de ladite composition et de préférence inférieure ou égale à 8%.

**15.** Composition selon l'une quelconque des revendications précédentes, **caractérisée par le fait que** le sel peroxygéné est présent dans une proportion pondérale comprise entre 20 et 70% et de préférence entre 30 et 60% par rapport au poids total de la composition.

**16.** Utilisation de la composition pulvérulente selon l'une quelconque des revendications précédentes pour la préparation d'une composition prête à l'emploi destinée à la décoloration des fibres kératiniques humaines et plus particulièrement des cheveux, par mélange au moment de l'emploi, de la dite poudre avec une composition aqueuse de peroxyde d'hydrogène ne titrant pas plus de 40 volumes.

**17.** Utilisation selon la revendication 16, **caractérisée par le fait que** le titre en peroxyde d'hydrogène de la composition aqueuse de peroxyde d'hydrogène varie de 2 à 40 volumes.

**18.** Utilisation selon l'une quelconque des revendications 16 ou 17, **caractérisée par le fait que** la composition aqueuse de peroxyde d'hydrogène contient :

(i) soit au moins un agent tensioactif dont au moins l'un est anionique ou non-ionique, dans une proportion pondérale totale comprise entre 0,1 et 10% et de préférence entre 0,1 et 5% par rapport au poids total de la composition aqueuse de peroxyde d'hydrogène;

(ii) soit au moins l'association de deux agents tensioactifs non-ioniques de HLB différents dont au moins l'un est inférieur à 5, dans une proportion pondérale totale comprise entre 1,5 et 40% et de préférence entre 1,5 et 20% par rapport au poids total de la composition aqueuse de peroxyde d'hydrogène;

(iii) soit au moins l'association d'un agent tensioactif non ionique de HLB inférieur ou égal à 5 et d'un agent tensioactif anionique, dans une proportion pondérale totale comprise entre 1 et 30% et de préférence entre 1,5 et 15% par rapport au poids total de la composition aqueuse de peroxyde d'hydrogène;

(iv) soit au moins un polymère épaississant de nature chimique quelconque, dans une proportion pondérale comprise entre 0,1 et 10% et de préférence èntre 0,1 et 5% par rapport au poids total de la composition aqueuse de peroxyde d'hydrogène;
ledit polymère étant éventuellement associé à un agent tensioactif présent dans une proportion pondérale comprise entre 0,1 et 10% et de préférence entre 0,1 et 5% par rapport au poids total de la composition aqueuse de peroxyde d'hydrogène.

**19.** Procédé de décoloration des fibres kératiniques humaines et plus particulièrement des cheveux, **caractérisé par le fait qu'**il consiste à mélanger, au moment de l'emploi, une composition pulvérulente telle que décrite à l'une quelconque des revendications 1 à 15, avec une composition aqueuse de peroxyde d'hydrogène ne titrant pas plus de 40 volumes et telle que décrite à l'une quelconque des revendications 16 à 18, à appliquer la composition de décoloration prête à l'emploi ainsi obtenue sur la zone des fibres kératiniques humaines sèches ou humides à décolorer, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

**20.** Dispositif à plusieurs compartiments, ou "kit", pour la décoloration des fibres kératiniques humaines et plus particulièrement des cheveux, **caractérisé par le fait qu'**il comporte au moins deux compartiments dont l'un contient une composition pulvérulente telle que décrite à l'une quelconque des revendications 1 à 15, et l'autre une composition aqueuse de peroxyde d'hydrogène ne titrant pas plus de 40 volumes.

**21.** Procédé de préparation d'une composition pulvérulente pour la décoloration des fibres kératiniques humaines et plus particulièrement des cheveux, **caractérisée par le fait qu'**elle consiste à mélanger simplement à la température de 25°C, le ou les agents réduisant le taux de poussière tels que décrits à l'une quelconque des revendications 1 à 7, au mélange préformé des autres composants de la composition pulvérulente telle que décrite à l'une quelconque des revendications 1 à 15.